(19) Europäisches Patentamt
European Patent Office
Office européen des brevets

(11) Publication number: **0 088 593**
**B1**

(12) # EUROPEAN PATENT SPECIFICATION

(45) Date of publication of patent specification: **27.05.87**

(21) Application number: **83301142.2**

(22) Date of filing: **03.03.83**

(51) Int. Cl.[4]: **C 07 D 401/04,**
C 07 D 403/04,
C 07 D 253/06, A 61 K 31/33,
C 07 D 491/04 // C07D241/18,
C07D241/16 ,(C07D491/04,
317:00, 221:00)

(54) **1,2,4-Triazine and pyrazine derivatives.**

(30) Priority: **05.03.82 US 354982**

(43) Date of publication of application:
**14.09.83 Bulletin 83/37**

(45) Publication of the grant of the patent:
**27.05.87 Bulletin 87/22**

(84) Designated Contracting States:
**AT BE CH DE FR GB IT LI LU NL SE**

(56) References cited:
**FR-A-2 391 202**
**US-A-3 979 516**
**US-A-4 081 542**

**The file contains technical information submitted after the application was filed and not included in this specification**

(73) Proprietor: **ELI LILLY AND COMPANY**
**307, East McCarty Street**
**Indianapolis Indiana 46285 (US)**

(72) Inventor: **Wong, David Taiwai**
**1640 Ridge Hill Lane**
**Indianapolis Indiana 46217 (US)**
Inventor: **Lacefield, William Bryant**
**636 Boulder Road**
**Indianapolis Indiana 46217 (US)**

(74) Representative: **Crowther, Terence Roger et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 088 593 B1**

## Description

This invention provides novel compounds which can be used as activators of gamma-aminobutyric acid (GABA) and benzodiazepine binding in the central nervous system of mammals.

GABA is recognized as a major inhibitory neurotransmitter. GABA receptors and benzodiazepine receptors were discovered in 1977. That there is interaction between GABA and benzodiazepine receptors was discovered in 1978. These findings provided the first biochemical evidence that some of the therapeutic effects of the benzodiazepines result from a facilitation of GABA receptor function.

Many clinical conditions are thought to arise, in part, from the imbalance between neurotransmission of GABA and those of other neurotransmitters. These conditions include Huntington's chorea, Parkinson's disease, spasticity, epilepsy, schizophrenia and tardive dyskinesia. Decreased GABA activity appears to contribute to the pathogenesis of these diseases. In addition, analgesia and satiety are thought to be regulated by GABA activity. Methods of modifying GABAergic neurotransmission are therefore desirable in order to modify these conditions.

Reduced GABA neuronal function can occur by the inhibition of GABA synthesis, by the blocking of the GABA receptors, or by the inhibition of chloride permeability. By reversing any or all of these functions, a therapeutic effect is possible. For instance, GABA agonists (which stimulate the GABA receptor), compounds which decrease GABA metabolism, and compounds which activate the GABA receptor by stimulating the benzodiazepine receptor have all been reported to inhibit a variety of induced seizure states. Several drugs, such as the benzodiazepines and progabide, have been found to be clinically effective as anticonvulsive agents, although many are limited or prevented in their use because of toxicity or secondary effects.

FR—A—2391202 and US—A—3979516 disclose 3-(cyclic)amino-5,6-diaryl-1,2,4-imazines having analgesic, antipyretic and/or NSAIDS activity, and US—A—4081542 describes 2-piperazinyl-5,6-diaryl-1,4-pyrazines with anorexic activity.

This invention provides novel compounds which demonstrate an ability to increase GABA and benzodiazepine binding and which also provide a therapeutic benefit in mammals having conditions derived from decreased GABA neuronal function but which avoid certain side effects and other undesirable attributes of compounds currently available for these disease states.

Specifically, the present invention provides compounds of the formula (I):

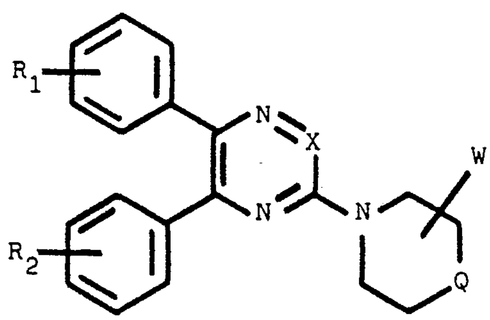

(I)

wherein

$R_1$ and $R_2$ are independently $C_1$—$C_3$ alkyl or chloro;

X is CH or N;

Q is oxygen or $—(CH_2)_n—$, where n is 0, 1, or 2;

W is a monovalent radical selected from hydrogen, $C_1$—$C_3$ alkyl, $C_1$—$C_3$ alkoxy, hydroxy, hydroxymethyl, —OCHO, —OCOA, $—OSO_2A$, or —COB, where A is $C_1$—$C_4$ alkyl, phenyl, phenoxy, amino, $C_1$—$C_3$ alkyl substituted phenyl, or mono- or di-$C_1$—$C_3$ alkylamino, and where B is $C_1$—$C_3$ alkoxy, amino, or mono- or di-$C_1$—$C_3$ alkylamino, or W is a divalent radical selected from =O, =NOH, or

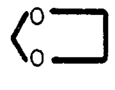,

and pharmaceutically acceptable salts thereof.

As used herein, the term "$C_1$—$C_3$ alkyl" includes methyl, ethyl, n-propyl, and isopropyl. The term "$C_1$—$C_4$ alkyl" includes methyl, ethyl, n-propyl, isopropyl, n-butyl, sec-butyl, and tert-butyl. The term "$C_1$—$C_3$ alkoxy" includes methoxy, ethoxy, n-propoxy, and isopropoxy.

The novel compounds of formula (I) referred to above can be prepared by methods known to the art using known starting materials. Specifically, the preparation of 3-amino-5,6-diaryl-1,2,4-triazines and 2-amino-5,6-diaryl-pyrazines by amine nucleophilic displacement of a labile group at the 3-position of 3-substituted-5,6-diaryl-1,2,4-triazines and at the 2-position of a 2-substituted-5,6-diaryl-pyrazines, is known.

Accordingly, in one aspect of the present invention there is provided a process for preparing a compound of the formula (I):

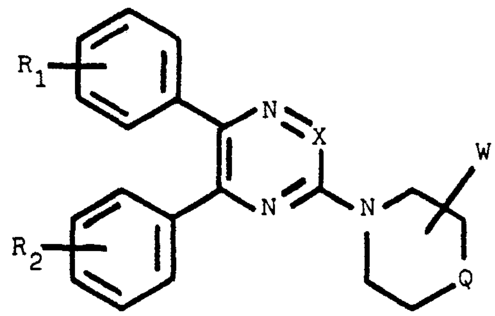

(I)

which comprises:

(a) reacting a precursor compound of formula (II):

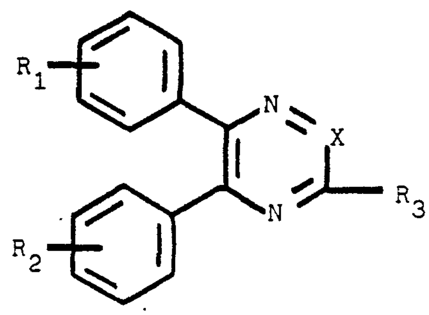

(II)

wherein $R_1$, $R_2$, and X are as defined above, and $R_3$ is a leaving group capable of undergoing nucleophilic displacement, with an amine of formula (III):

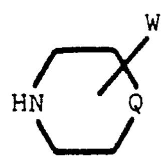

(III)

wherein W and Q are as defined above, and if appropriate optionally alkylating or esterifying a product of formula (I) in which W is hydroxyl; or

(b) reacting a compound of formula (I) in which W is a group of formula:

$$-O-\overset{\overset{O}{\|}}{C}-OPhenyl$$

with ammonia or an amine of formula $HNR^{17}R^{18}$ where $R^{17}$ is $C_1$—$C_3$ alkyl and $R^{18}$ is hydrogen or $C_1$—$C_3$ alkyl so as to prepare a compound of formula (I) in which W is a group of formula —OCOA where A is amino or a mono- or di-$C_1$—$C_3$ alkylamino group.

Preferred 3-substituted-5,6-diaryl-1,2,4-triazine and 2-substituted-5,6-diaryl-pyrazine compounds of formula (II) are ones wherein the leaving group $R_3$ is chloro, methoxy, or methylmercapto. It will be recognized by those skilled in the art, however, that numerous other leaving groups can be used. Groups capable of undergoing the required nucleophilic displacement include, for example, $NO_2$, halo, $C_1$—$C_4$ alkylmercapto, sulfonates such as tosylate, mesylate and brosylate, $C_1$—$C_4$ alkoxy, and azido.

Appropriate triazine and pyrazine precursers can be made using known procedures. The preparation of 5,6-diaryl-1,2,4-triazines is described by John G. Erickson in "The 1,2,3- and 1,2,4-Triazines, Tetrazines and Pentazines." The Chemistry of Heterocyclic Compounds, Vol. 10, Interscience Publishers, Inc., New York, N.Y., 1956, Chapter II, pp. 44—84.

To prepare 3-chloro-5,6-diaryl-1,2,4-triazines, for example, appropriate benzil starting materials are condensed with semicarbazide or its hydrochloride to provide 3-hydroxy-5,6-diaryl-1,2,4-triazine intermediates. The 3-hydroxytriazines are converted to the corresponding 3-chlorotriazines by reaction with phosphorous oxychloride.

Corresponding 3-methoxy-5,6-diaryl-1,2,4-triazines are prepared by methanolysis of the 3-chlorotriazines under basic conditions. Other 3-alkoxy precursors are similarly prepared.

Triazine compounds of formula (II) wherein $R_3$ is methylmercapto can be prepared by alkylating 5,6-diaryl-1,2,4-triazine-3-thiols with methyl iodide under basic conditions. The 3-thiols are prepared by condensation of benzils with thiosemicarbazide. For example, the reaction of 4,4'-dichlorobenzil with thiosemicarbazide provides the 5,6-bis(4-chlorophenyl)-1,2,4-triazine-3-thiol intermediate, which can be converted to the 3-methylmercapto derivative. Alternatively, 3-methylmercapto-5,6-diaryl-1,2,4-thiazine compounds can be prepared by condensing benzils with S-methylthiosemicarbazide. $C_2$—$C_3$ alkylmercapto precursors are prepared similarly.

The pyrazine compounds can be prepared in an analogous manner. Yolanda T. Pratt in Heterocyclic Compounds, Volume 6, Part 2, John Wiley and Sons, Inc., New York, N.Y., 1957, Chapter 9, pp. 377—454, describes the preparation of 5,6-disubstituted-2-hydroxypyrazines from the appropriately substituted diones and glycine amide, as well as the subsequent transformation of the 2-hydroxypyrazines to the corresponding 2-chloro analogs by reaction with phosphorous oxychloride. The 2-chloro intermediates are reacted with amines via nucleophilic displacement in the same manner as previously described for the triazine compounds.

The benzils required for the triazine and pyrazine intermediates are prepared by the oxidation of benzoins obtained from aromatic aldehydes via reaction with cyanide ion, i.e., the classic benzoin condensation [See Organic Reactions *4*, 269 (1948)]. The resultant benzoins are oxidized to benzils with copper sulfate in pyridine as described by Clarke and Driger, Organic Synthesis, Coll. Vol. I, 87 (1941), for example.

Unsymmetrical benzils are obtained from mixed benzoins which arise when dissimilar aldehydes are condensed. The benzil compounds required for the preparation of the starting materials and intermediate triazines and pyrazines are represented by the formula

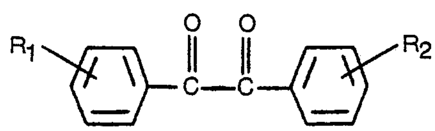

wherein $R^1$ and $R^2$ are described hereinabove. When $R^1$ and $R^2$ represent different groups the depicted benzils are unsymmetrical. The use of unsymmetrical benzil starting materials may result in the preparation of isomer mixtures of triazines or pyrazines. For example, the condensation of 4-methyl-4'-chlorobenzil with thiosemicarbazide provides a mixture of 5-(4-methylphenyl)-6-(4-chlorophenyl)-1,2,4-triazine-3-thiol and 6-(4-methylphenyl)-5-(4-chlorophenyl)-1,2,4-triazine-3-thiol.

It will be recognized by those skilled in the art that isomeric mixtures of triazines or pyrazines are separable by methods such as fractional crystallization or chromatography. The isomer separation may be effected upon intermediate mixtures or delayed until the final product stage.

Many of the reactant amines, represented by the formula (III)

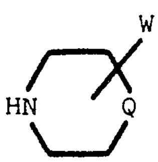

(III)

wherein Q and W are as previously defined, are commercially available. Others are derivatives of commercially available amines and those skilled in the art may perform such derivatizations using the conventional methods of acylation, alkylation, aminolysis, esterification, hydrolysis, etc. Although in some cases derivatization may be performed prior to the condensation with the triazine intermediate, those skilled in the art will recognize that it is frequently easier or mandatory to perform the derivatization of the amine after the condensation with the triazine intermediate since the presence of the secondary amine functionality may hinder or prevent the desired derivatization. Still other amines are prepared by the methods known to the art, i.e., the alkylation of ammonia, the reduction of cyanides, nitro compounds and oximes, reductive alkylation, the Curtius reaction, the Gabriel amine synthesis, the Hofmann reaction, the Leuckart reaction, the Schmid reaction, etc., followed by condensation and/or derivatization in the proper order as described above.

The reaction between the precursor of formula (II) and the amine of formula (III) may be carried out by mixing the two reactants at a temperature of about 80 to 150°C, preferably 100 to 150°C. Usually the reaction is most conveniently carried out at the reflux temperature. If the boiling point of the amine is less than 100°C, it may be desirable to carry the reaction out in a sealed system so that the temperature can be elevated to the preferred range, where the reaction takes place more quickly. If desired, the reaction can be carried out in a nonreactive organic solvent, such as an alcohol, benzene, dioxane, pyridine, toluene, chloroform, xylene, and the like. The nucleophilic amines are used in molar equivalent amounts or in excess. When the leaving group $R_3$ is halo, it is convenient to use an excess of the nucleophilic amine, since the amine serves as a halo-acid scavenger. Thus, the molar ratio of amine of formula (III) to precursor of formula (II) used may typically be in the range of 1:1 to 5:1, for example. If the amount of amine used is an

economic consideration, organic or inorganic scavengers such as pyridine, triethylamine, sodium carbonate and the like can be used instead of excess amine.

As previously mentioned, it may frequently be desirable to derivatize the amine group (III) after it is condensed with the triazine or pyrazine. The present invention specifically encompasses, for example, the esterification or alkylation of a compound of formula (I) wherein W is hydroxyl.

The alkylation or esterification of the product of the nucleophilic displacement in which W is hydroxyl can be effected using techniques known *per se* in the art. For instance, the alkylation can be effected in the presence of a strong base such as sodium hydride using an alkylating agent of formula $R_{10}L$ where L is a leaving group, for instance a halo radical such as iodo, and $R_{10}$ is $C_1$—$C_3$ alkyl. Any inert organic solvent which is unreactive to the reagents may be utilized. As one example, hydrocarbon solvents such as toluene may be used.

The esterification can be effected using an appropriate monocarboxylic acid or activated derivative thereof such as an ester, acid halide, preferably the acid chloride, or anhydride thereof. In general, such reagents may be depicted by the structure:

$$A—\overset{\overset{O}{\|}}{C}—Z$$

where A is as defined above and Z is hydroxyl, halo, —$OR_{11}$ where $R_{11}$ is a $C_1$—$C_4$ alkyl group, or is —O—CO—A. The invention also includes reaction of the alcohol of formula (I) with sulfonic acid derivatives, such as A—$SO_2$—M, where A is as defined above and M is a leaving group such as halo. Thus, for example, a compound of formula (I) wherein W is hydroxyl can be reacted with an anhydride of a $C_2$—$C_5$ carboxylic acid, such as acetic anhydride, or with an acyl halide such as pivaloyl chloride, benzoyl chloride, or phenylchloroformate, or with a sulfonyl halide such as methane, sulfonyl chloride or tosyl chloride to provide compounds of formula (I) wherein in W is —O—CO—H, —O—CO—A, or —O—$SO_2$—A.

A preferred embodiment of this novel invention consists of those compounds in which both substituted aryl groups are the same (Formula I, $R_1 = R_2$). A second preferred embodiment is where the substituents on the aryl rings are in the 4-position, especially 4-chlorophenyl and 4-methylphenyl derivatives. With respect to the substituent in the 3-position of the triazine ring or the 2-position of the pyrazine ring, the preferred groups are those in which a hydroxy or especially an acyloxy group is the substituent on the amine ring, derivatives of the 4-hydroxypiperidine ring (Q is $CH_2$) being most preferred.

Representative of the compounds provided by this invention are the following:

1-[5,6-bis(4-methylphenyl)-pyrazin-2-yl]-4-piperidinol,
1-[5,6-bis(4-methylphenyl)-1,2,4-triazin-3-yl]-4-piperidinol,
1-[5-(4-methylphenyl)-6-(4-chlorophenyl)-1,2,4-triazin-3-yl]-4-piperidinol,
1-[5-(4-chlorophenyl)-6-(4-methylphenyl)-1,2,4-triazin-3-yl]-4-piperidinol,
1-[5,6-bis(4-chlorophenyl)-1,2,4-triazin-3-yl]-3-pyrrolidinol,
1-[5-(2-methylphenyl)-6-(4-methylphenyl)-1,2,4-triazin-3-yl]-3-piperidinol,
1-[5-(2-chlorophenyl)-6-(3-ethylphenyl)-1,2,4-triazin-3-yl]-4-piperidinol,
1-[5-(4-isopropylphenyl)-6-(3-chlorophenyl)-1,2,4-triazin-3-yl]-4-piperidinol, benzoate (ester),
1-[5,6-bis(4-methylphenyl)-1,2,4-triazin-3-yl]-4-piperidinol, acetate (ester),
1-[5,6-bis(4-chlorophenyl)-1,2,4-triazin-3-yl]-3-piperidinol, acetate (ester),
1-[5-(3-methylphenyl)-6-(3-n-propylphenyl)-1,2,4-triazin-3-yl]-4-piperidinol, t-butanoate (ester),
1-[5-(2-ethylphenyl)-6-(3-chlorophenyl)-pyrazin-2-yl]-3-piperidinol, propionate (ester),
5,6-bis(2-chlorophenyl)-3-piperidinyl-1,2,4-triazine,
3-(4-ethoxy-1-piperidinyl)-5,6-bis(3-methylphenyl)-1,2,4-triazine,
2-morpholino-5,6-bis(3-chlorophenyl)-pyrazine,
1-[5,6-bis(4-isopropylphenyl)-1,2,4-triazin-3-yl]-4-piperidinone,
1-[5-(3-chlorophenyl)-6-(2-n-propylphenyl)-1,2,4-triazin-3-yl]-3-pyrrolidinone,
1-[5,6-bis(3-ethylphenyl)-pyrazin-2-yl]-4-piperidinol, diethyl carbamate (ester),
1-[5,6-bis(4-chlorophenyl)-1,2,4-triazin-3-yl)-3-piperidinol, methane sulfonate (ester),
1-[5,6-bis(4-n-propylphenyl)-pyrazin-2-yl]-4-piperidinol, propyl carbamate (ester),
1-[5,6-bis(4-chlorophenyl)-pyrazin-2-yl]-4-piperidinone, oxime,
1-[5-(2-methylphenyl)-6-(3-ethylphenyl)-1,2,4-triazin-3-yl]-3-piperidinemethanol,
1-[5-(3-chlorophenyl)-6-(4-isopropylphenyl)-pyrazin-2-yl]-3-piperidinecarboxylic acid, ethyl ester,
1-[5-(3-isopropylphenyl)-6-(4-isopropylphenyl)-pyrazin-2-yl]-3-pyrrolidinecarboxylic acid, N,N-diethylamide,
1-[5,6-bis(3-methylphenyl)-1,2,4-triazin-3-yl]-4-piperidinol, formate (ester),
1-[5-(2-chlorophenyl)-6-(3-chlorophenyl)-1,2,4-triazin-3-yl]-4-piperidinecarboxylic acid, N-isopropyl amide,
1-[5,6-bis(2-methylphenyl)-pyrazin-2-yl]-3-piperidinol, 4-propylbenzene sulfonate, ester,
8-[5-(4-n-propylphenyl)-6-(3-chlorophenyl)-1,2,4-triazin-3-yl]-1,4-dioxa-8-azaspiro(4.5)decane,
1-[5,6-bis(3-n-propylphenyl)-1,2,4-triazin-3-yl]-hexahydro-1H-azepine,
1-[5,6-bis(4-ethylphenyl)-1,2,4-triazin-3-yl]-3-pyrrolidinol, propane sulfonate (ester),

1-[5-(2-methylphenyl)-6-(3-isopropylphenyl)-pyrazin-2-yl]-4-propyl-hexahydro-1H-azepine,
1-[5-(4-isopropylphenyl)-6-(3-methylphenyl)-1,2,4-triazin-3-yl]-3-piperidinemethanol,
1-[5-(2-ethylphenyl)-6-(2-methylphenyl)-pyrazin-2-yl]-3-hydroxy-hexahydro-1H-azepine, n-butanoate (ester),
3-(3-propoxy-1-pyrrolidinyl)-5-(2-propylphenyl)-6-(4-propylphenyl)-1,2,4-triazine,
3-morpholino-5-(4-ethylphenyl)-6-(4-isopropylphenyl)-1,2,4-triazine,
8-[5-(4-propylphenyl)-6-(4-methylphenyl)-1,2,4-triazin-3-yl]-1,4-dioxa-8-azaspiro(4.5)decane,
carbonic acid, 1-[5-(2-ethylphenyl)-6-(3-ethylphenyl)-1,2,4-triazin-3-yl]-4-piperidinyl phenyl ester,
1-[5-(4-ethylphenyl)-6-(4-n-propylphenyl)-pyrazin-2-yl]-3-piperidinone,
1-[5-(4-n-propylphenyl)-6-(3-ethylphenyl)pyrazin-2-yl]-3-pyrrolidinecarboxamide,
carbonic acid, 1-[5-(4-isopropylphenyl)-6-(2-ethylphenyl)-pyrazin-2-yl]-3-pyrrolidinyl phenyl ester,
1-[5-(3-isopropylphenyl)-6-(3-propylphenyl)pyrazin-2-yl]-4-piperidinol, acetate (ester), and
1-[5-(3-propylphenyl)-6-(4-isopropylphenyl)pyrazin-2-yl]-3-piperidinol, formate (ester).

The compounds provided by this invention are effective over a wide dosage range, the actual dose administered being dependent on such factors as the particular compound being used, the condition being treated and the type and size of mammal being treated. However, a single dosage will normally fall within the range of 1—500 mg in the treatment of adult humans, it being understood that the single dosage may be repeated up to four or five times per day.

The active compounds provided by the present invention will normally be administered orally or by injection and, for this purpose, said compounds or salts thereof, if applicable, will usually be utilized in the form of a pharmaceutical composition. Such compositions are prepared in a manner well known in the pharmaceutical art and normally comprise at least one active compound or salt provided by the invention in association with a pharmaceutically-acceptable carrier therefor. In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Some examples of suitable carriers are lactose, dextrose, sucrose, sorbitol, mannitol, starches, gum acacia, calcium phosphate, alginates, tragacanth, gelatin, syrup, methyl cellulose, methyl- and propyl-hydroxybenzoate, talc, magnesium stearate or mineral oil. The compositions of the invention may, as is well known in the art, be formulated so as to provide quick, sustained or delayed release of the active ingredient after administration to the patient.

Depending on the route of administration, the foregoing compositions may be formulated as tablets, capsules or suspensions for oral use and injection solutions for parenteral use. Preferably the compositions are formulated in a dosage unit form.

Accordingly, in one aspect of the invention there is provided a pharmaceutical composition which comprises a compound of formula (I) or a pharmaceutically-acceptable salt thereof, associated with a pharmaceutically-acceptable carrier therefor.

In a further aspect of the invention there is provided a method of preparing a pharmaceutical composition as described above which comprises admixing a compound of formula (I), or a pharmaceutically-acceptable salt thereof, with a pharmaceutically-acceptable carrier therefor.

In yet a further aspect of the invention there is provided a method of treating mammals, particularly humans, which comprises administering a chemotherapeutically effective amount of a compound of formula (I), or a pharmaceutically-acceptable salt thereof, to the afflicted mammal.

The following examples illustrate the preparations of the novel compounds of this invention.

Preparation 1

Preparation of 3-methylmercapto-5,6-bis(4-methylphenyl)-1,2,4-triazine

A. The above compound was prepared as taught in U.S. patent No. 4,013,654 (Example 3) or No 4,018,923 (Example 3) by methylating the intermediate 3-mercapto derivative obtained from the reaction of thiosemicarbazide and 4,4′-dimethylbenzil.

B. The above compound was alternatively prepared in the following manner: To a solution of 500 g. (2.09 m.) of 4,4′-dimethylbenzil in 3 liters of methanol was added 512 g. (2.2 m.) of S-methyl-thiosemicarbazide hydroiodide (prepared from the action of methyl iodide on thiosemicarbazide in methanol), and 184.8 g. (2.2 m.) of sodium bicarbonate. After stirring for 18 hours at room temperature, the yellow precipitate was separated by filtration, washed with one liter of water, and oven dried for 12 hours, yielding 679.3 g. (100% yield) of the desired product, m.p. 169—170°C.

| | | | |
|---|---|---|---|
| Analysis: | $C_{18}H_{17}N_3S$; | | |
| Calc: | C, 70.33; | H, 5.57; | N, 13.67; |
| Found: | C, 70.04; | H, 5.75; | N, 13.71. |

Preparations 2—4

The following compounds were prepared by the method of Preparation 1(B) using the appropriate substituted benzil and S-methyl-thiosemicarbazide.

3-methylmercapto-5,6-bis[2-methylphenyl]-1,2,4-triazine, m.p. 109.5—110.0°C.

Analysis: $C_{18}H_{17}N_3S$;
Calc: C, 70.33; H, 5.57; N, 13.67;
Found: C, 70.57; H, 5.31; N, 13.48.

3-methylmercapto-5,6-bis[3-methylphenyl]-1,2,4-triazine, m.p. 87—89°C.

3-methylmercapto-5,6-bis[4-ethylphenyl]-1,2,4-triazine, m.p. 90—91°C.

Analysis: $C_{20}H_{21}N_3S$;
Calc: C, 71.61; H, 6.31; N, 12.53; S, 9.56;
Found: C, 71.31; H, 6.16; N, 12.31; S 9.84.

Preparation 5

Preparation of 3-methylmercapto-5,6-bis[4-chlorophenyl]-1,2,4-triazine

Following the procedures as taught in U.S. Patent No. 4,013,654 (Example 3) and No. 4,018,923 (Example 3), 3-mercapto-5,6-bis(4-chlorophenyl)-1,2,4-triazine was treated with methyl iodide in the presence of sodium hydroxide and ethanol to give 3-methylmercapto-5,6-bis[4-chlorophenyl]-1,2,4-triazine, m.p. 125.5—128°C. (crystallized from 2B alcohol).

Analysis: $C_{16}H_{11}N_3Cl_2S$;
Calc: C, 55.18; H, 3.18; N, 12.07;
Found: C, 55.57; H, 3.17; N, 12.29.

Preparation 6

Preparation of 3-chloro-5,6-bis(4-methylphenyl)-1,2,4-triazine

In a manner similar to that described for the bis(4-chlorophenyl) analog (see U.S. patent 3,989,831, Example 3), 100.0 g. (0.36 m.) of 3-hydroxy-5,6-bis-(4-methylphenyl)-1,2,4-triazine were allowed to reflux in 100 ml. of phosphorus oxychloride for about two hours. After cooling, the solution was slowly poured onto crushed ice. The resulting precipitate was extracted into ethyl acetate which was then washed twice with water and once with 2% aqueous sodium hydroxide. The organic solution was dried over anhydrous sodium sulfate, filtered, and evaporated *in vacuo*. The resulting oil was triturated with Skelly B/cyclohexane and filtered. The filtrate was treated with decolorizing carbon, filtered, and evaporated to give 37.4 g. (35.2% yield) of the title compound, m.p. about 126.5—129.5°C.

Analysis: $C_{17}H_{14}N_3Cl$;
Calc: C, 69.04; H, 4.77; N, 14.21; Cl, 11.99;
Found: C, 68.85; H, 4.99; N, 14.04; Cl, 11.70.

Preparation 7

Preparation 2-hydroxy-5,6-bis(4-methylphenyl)pyrazine

To a refluxing solution of 85.6 g. (0.36 m.) of 4,4'-dimethylbenzil and 40 g. (0.36 m.) of glycinamide hydrochloride in one liter of methanol was added 64 ml. (0.8 m.) of a 12.5N solution of sodium hydroxide over a 75 minute period. The solution was then refluxed for one hour. After cooling, 50 ml. of 12N hydrochloric acid was added, followed by the addition of 40 g. of dry potassium bicarbonate and about 10 ml. of water. The resulting precipitate was filtered off and crystallized from n-butanol. The filtrate of crystallization was evaporated to give 2-hydroxy-5,6-bis(4-methylphenyl)-pyrazine, m.p. 250—254°C.

Analysis: $C_{18}H_{16}N_2O$;
Calc: C, 78.24; H, 5.84; N, 10.14;
Found: C, 78.13; H, 5.56; N, 9.90.

Preparation 8

Preparation of 2-chloro-5,6-bis(4-methylphenyl)pyrazine

A solution of 62.6 g. (0.226 m.) of 2-hydroxy-5,6-bis(4-methylphenyl)pyrazine (Preparation 7) and 250 ml. of phosphorous oxychloride was allowed to reflux overnight. The solution was then poured into 200 ml. of ice water and 300 ml. of ether. After filtration, the layers were separated. The aqueous layer was made basic with 28% ammonium hydroxide and then extracted with ethyl acetate. The ethyl acetate solution was dried, filtered, and concentrated. The concentrate was triturated with Skelly B and filtered. The filtrate was evaporated to give 2-chloro-5,6-bis(4-methylphenyl)-pyrazine.

Analysis: $C_{18}H_{15}N_2Cl$;
Calc: C, 73.34; H, 5.13; N, 9.50;
Found: C, 73.21; H, 5.68; N, 9.11.

Example 1

Preparation of 1-[5,6-bis(4-methylphenyl)-1,2,4-triazin-3-yl]-4-piperidinol

A solution of 150 g. (0.488 m.) of 3-methyl-mercapto-5,6-bis(4-methylphenyl)-1,2,4-triazine was heated at about 150°C. for 20 hours in 100 g. (0.99 m.) of 4-hydroxypiperidine. The solution was added to crushed ice, a small amount of ethanol was added, and the suspension was stirred for 2 hours. The yellow solid was isolated by filtration and dried. On crystallization from 550 ml. 2B ethanol and 100 ml. water, 167 g. (95.1% yield) of the desired product was obtained, m.p. 167—168.5°C.

Analysis: $C_{22}H_{24}N_4O$;
Calc: C, 73.31; H, 6.71; N, 15.54;
Found: C, 73.34; H, 6.71; N, 15.50.

Example 2

Preparation of 1-[5,6-bis(4-ethylphenyl)-1,2,4-triazin-3-yl]-4-piperidinol

Following the procedure in Example 1, 38.45 g. (0.115 m.) of 3-methylmercapto-5,6-bis(4-ethylphenyl)-1,2,4-triazine were reacted with 23.22 g. (0.23 m.) of 4-hydroxypiperidine to give 29.1 g. (65.3% yield) of the desired product, m.p. 164.5—166.5°C.

Analysis: $C_{24}H_{28}N_4O$;
Calc: C, 74.20; H, 7.26; N, 14.42;
Found: C, 74.21; H, 7.22; N, 14.36.

Example 3

Preparation of 1-[5,6-bis(2-methylphenyl)-1,2,4-triazin-3-yl]-4-piperidinol

Following the procedure in Example 1, 29 g. (0.095 m.) of 3-methylmercapto-5,6-bis(2-methylphenyl)-1,2,4-triazine were reacted with 46.5 g. (0.46 m.) of 4-hydroxypiperidine to give 27.8 g. (81.3% yield) of the desired product, m.p. 155.0—155.5°C.

Analysis: $C_{22}H_{24}N_4O$;
Calc: C, 73.31; H, 6.71; N, 15.54;
Found: C, 73.19; H, 6.44; N, 15.25.

Example 4

Preparation of 1-[5,6-bis(3-methylphenyl)-1,2,4-triazin-3-yl]-4-piperidinol

Following the procedure in Example 1, 44.3 g. (0.14 m.) of 3-methlmercapto-5,6-bis(3-methylphenyl)-1,2,4-triazine were reacted with 29.2 g. (0.29 m.) of 4-hydroxypiperidine to give the desired product, m.p. 60°C.

Analysis: $C_{22}H_{24}N_4O$;
Calc: C, 73.31; H, 6.71; N, 15.54;
Found: C, 73.51; H, 6.96; N, 15.35.

Example 5

Preparation of 1-[5,6-bis(4-chlorophenyl)-1,2,4-triazin-3-yl]-4-piperidinol

Following the procedure in Example 1, 18.4 g. (0.053 m.) of 3-methylmercapto-5,6-bis(4-chlorophenyl)-1,2,4-triazine were reacted with 13.13 g. (0.13 m.) of 4-hydroxypiperidine to give 3.51 g. (16.6% yield) of the desired product, m.p. 102—105°C.

Analysis: $C_{20}H_{28}N_4OCl_2$;
Calc: C, 59.86; H, 4.52; N, 13.96;
Found: C, 59.71; H, 4.52; N, 13.68.

Examples 6—8

Preparation of acetate derivatives

Some of the hydroxypiperidine compounds of this invention were acetylated using various conventional techniques.

For example, 20 g. (0.055 m.) of 1-[5,6-bis(4-methylphenyl)-1,2,4-triazin-3-yl]-4-piperidinol were refluxed with 70 ml. of pyridine and 160.9 g. of acetic anhydride for 4 hours. After cooling and concentration, ethanol and water were added, and the solution evaporated. Recrystallization from ethanol-water yielded 21.8 g. (98.2% yield) of 1-[5,6-bis-(4-methylphenyl)-1,2,4-triazin-3-yl]-4-piperidinol, acetate (ester), m.p. 124—126°C.

Analysis: $C_{24}H_{26}N_4O_2$;
Calc: C, 71.62; H, 6.51; N, 13.92;
Found: C, 71.81; H, 6.31; N, 13.78.

In the same manner was prepared 1-[5,6-bis[4-chlorophenyl)-1,2,4-triazin-3-yl]-4-piperidinol, acetate (ester)

Analysis: $C_{22}H_{20}N_4O_2Cl_2$;
Calc: C, 59.60; H, 4.55; N, 12.64;
Found: C, 59.82; H, 4.68; n, 12.43.

In the same manner was prepared 1-[5,6-bis(2-methylphenyl)-1,2,4-triazin-3-yl]-4-piperidinol, acetate (ester)

Analysis: $C_{24}H_{26}N_4O_2$;
Calc: C, 71.62; H, 6.51; N, 13.92;
Found: C, 71.85; H, 6.70; N, 13.91.

Example 9

Preparation of 1-[5,6-bis(4-methylphenyl)-1,2,4-triazin-3-yl]-4-piperidinol, propanoate (ester)

According to the same general procedure for acetate preparation, 10.0 g. (0.027 m.) of 1-[5,6-bis(4-methylphenyl)-1,2,4-triazin-3-yl]-4-piperidinol, 165.1 g. of propionic anhydride, and 35 ml. of pyridine were reacted to give 8.8 g. (78.3% yield) of the desired product, m.p. 136—138°C.

Analysis: $C_{25}H_{28}N_4O_2$;
Calc: C, 72.09; H, 6.78; N, 13.45;
Found: C, 72.35; H, 6.50; N, 13.56.

Example 10

Preparation of 1-[5,6-bis(4-methylphenyl)-1,2,4-triazin-3-yl]-4-piperidinol, pivaloate (ester)

To 40 ml. of pyridine was added 5.0 g. (0.014 m.) of 1-[5,6-bis(4-methylphenyl)-1,2,4-triazin-3-yl]-4-piperidinol followed by the dropwise addition of 39.3 g. (0.328 m.) of pivaloyl chloride. The solution was heated to reflux for 4 hours. On cooling, the solution was poured onto crushed ice and extracted into ethyl acetate. The organic solution was washed twice with 5% aqueous sodium bicarbonate, once with water, dried with anhydrous sodium sulfate, filtered and concentrated. The concentrate was treated with hot ethyl acetate and decolorizing carbon. After filtration the solution was concentrated to an oil. The oil was chromatographed to provide the pure desired product, m.p. 130—132°C.

Analysis: $C_{27}H_{32}N_4O_2$;
Calc: C, 72.94; H, 7.26; N, 12.60;
Found: C, 72.92; H, 7.14; N, 12.46.

Example 11

Preparation of 1-[5,6-bis(4-methylphenyl)-1,2,4-triazin-3-yl]-4-piperidinol, benzoate (ester)

Following the procedure in Example 10, 5.0 g. (0.138 m.) of 1-[5,6-bis(4-methylphenyl)-1,2,4-triazin-3-yl]-4-piperidinol were allowed to react with 70 g. (0.5 m.) of benzoyl chloride in 35 ml. of pyridine to give 2.6 g. (40.3% yield) of the desired product, m.p. 171—173°C.

Analysis: $C_{29}H_{28}N_4O_2$;
Calc: C, 74.98; H, 6.08; N, 12.06;
Found: C, 74.66; H, 6.06; N, 11.88.

Example 12

Preparation of 1-[5,6-bis(4-methylphenyl)-1,2,4-triazin-3-yl]-3-pyrrolidinol

A solution of 8.7 g. (0.029 m.) of 3-chloro-5,6-bis(4-methylphenyl)-1,2,4-triazine and 5.05 g. (0.058 m.) of 3-pyrrolidinol in 250 ml. of chloroform were allowed to reflux overnight. The solution was then poured onto crushed ice which was then extracted with 600 ml. of methylene chloride. The organic layer was washed once with 600 ml. of water, then dried over anhydrous sodium sulfate, filtered and evaporated. The residue was crystallized from ethanol-water to give 9.3 g. (92.4% yield) of the desired compound, m.p. 147—150°C.

Analysis: $C_{21}H_{24}N_4O$;
Calc: C, 72.39; H, 6.94; N, 16.08;
Found: C, 72.45; H, 6.70; N, 15.83.

Example 13

Preparation of 1-[5,6-bis(4-methylphenyl)-1,2,4-triazin-3-yl]-3-pyrrolidinol, acetate (ester)

The reaction of 4.91 g. (0.014 m.) of 1-[5,6-bis(4-methylphenyl)-1,2,4-triazin-3-yl]-3-pyrrolidinol with acetic anhydride according to the procedure of Example 6 gave 4.9 g. (89.3% yield) of the desired product.

Analysis: $C_{23}H_{28}N_4O_2$;
Calc: C, 71.11; H, 6.23; N, 14.42;
Found: C, 70.86; H, 6.32; N, 14.12.

Example 14

Preparation of 5,6-bis(4-methylphenyl)-3-(1-piperidinyl)-1,2,4-triazine

The reaction of 15.0 g. (0.05 m.) of 3-chloro-5,6-bis(4-methylphenyl)-1,2,4-triazine with 7.0 g. (0.08 m.) of piperidine according to the procedure in Example 12 gave 8.6 g. (49.2% yield) of the desired product, m.p. 141—143°C.

Analysis: $C_{22}H_{24}N_4$;
Calc: C, 76.71; H, 7.02; N, 16.27;
Found: C, 76.44; H, 7.10; N, 15.98.

Example 15

Preparation of 1-[5,6-bis(4-methylphenyl)-1,2,4-triazin-3-yl)-hexahydro-1H-azepine

The reaction of 8.0 g. (0.027 m.) of 3-chloro-5,6-bis(4-methylphenyl)-1,2,4-triazine with 5.35 g. (0.054 m.) of hexamethylenimine according to the procedure in Example 12 gave 6.0 g. (62.5% yield) of the desired product, m.p. 162.5—164°C.

Analysis: $C_{23}H_{26}N_4$;
Calc: C, 77.06; H, 7.31; N, 15.63;
Found: C, 76.83; H, 7.44; N, 15.39.

Example 16

Preparation of 3-morpholino-5,6-bis(4-methylphenyl)-1,2,4-triazine

The reaction of 7.5 g. (0.024 m.) of 3-methylmercaptao-5,6-bis(4-methylphenyl)-1,2,4-triazine with 75 ml. of morpholine according to the procedure in Example 1 gave 4.7 g. (55.7% yield) of the desired product, m.p. 190—192.5°C.

Analysis: $C_{21}H_{22}N_4O$;
Calc: C, 72.81; H, 6.40; N, 16.17;
Found: C, 73.08; H, 6.12; N, 15.98.

Example 17

Preparation of 1-[5,6-bis(4-methylphenyl)-1,2,4-triazin-3-yl]-3-piperidinol

The reaction of 15.0 g. (0.05 m.) of 3-chloro-5,6-bis(4-methylphenyl)-1,2,4-triazine with 10.0 g. (0.1 m.) of 3-hydroxypiperidine according to the procedure in Example 12 gave 15.7 g. (87.2% yield) of the desired product, m.p. 122—125°C.

Analysis: $C_{22}H_{24}N_4O$;
Calc: C, 73.31; H, 6.71; N, 15.54;
Found: C, 73.19; H, 7.00; N, 15.74.

Example 18

Preparation of 1-[5,6-bis(4-methylphenyl)-1,2,4-triazine-3-yl]-3-piperidinol, acetate (ester)

The product of Example 17 was reacted with acetic anhydride according to the procedure of Example 6 to provide the acetate derivative. 89.7% yield, m.p. 162—163.5°C.

Analysis: $C_{24}H_{26}N_4O_2$;
Calc: C, 71.62; H, 6.51; N, 13.92;
Found: C, 71.42; H, 6.73; N, 13.70.

Example 19

Preparation of 5,6-bis(4-methylphenyl)-3-(4-methyl-1-piperidinyl)-1,2,4-triazine

Following the procedure of Example 12, 8.0 g. (0.027 m.) of 3-chloro-5,6-bis(4-methylphenyl)-1,2,4-triazine were reacted with 5.35 g. (0.054 m.) of 4-methylpiperidine to give 5.3 g. (54.9% yield) of the desired product, m.p. 140—142°C.

Analysis: $C_{23}H_{24}N_4$;
Calc: C, 77.50; H, 6.79; H, 15.72;
Found: C, 77.80; H, 7.00; N, 15.64.

Example 20

Preparation of 3-(4-methoxy-1-piperidinyl)-5,6-bis(4-methylphenyl)-1,2,4-triazine

To 1.3 grams of a 50% sodium hydride dispersion in oil in 180 ml. dry toluene was added 10.0 g. (0.27 m.) of 1-[5,6-bis(4-methylphenyl)-1,2,4-triazin-3-yl]-4-piperidinol. With stirring, 4.3 g. (0.03 m.) of methyl iodide were introduced to the reaction mixture and the reaction was brought to reflux. After cooling, an additional equivalent (4.3 g.) of methyl iodide was added and the reaction mixture was stirred at room temperature overnight. The solution was added to ice water and extracted with ethyl acetate. The extract was washed with water, 10% sodium hydroxide, and water, dried over anhydrous sodium sulfate and evaporated to dryness. Silica gel chromatography and crystallization from hexane gave 3.3 g. (31.8% yield) of the desired product, m.p. 120—121°C.

Analysis: $C_{23}H_{26}N_4O$;
Calc: C, 73.77; H, 7.00; N, 14.96;
Found: C, 73.66; H, 6.86; N, 14.75.

Example 21

Preparation of 3-(4-ethoxy-1-piperidinyl)-5,6-bis(4-methylphenyl)-1,2,4-triazine

Repeating Example 20, but using ethyl iodide in place of methyl iodide, the title product was obtained in about a 3% yield, m.p. 124—126°C.

Analysis: $C_{24}H_{28}N_4O$;
Calc: C, 74.20; H, 7.26; N, 14.42;
Found: C, 74.10; H, 7.00; N, 14.47.

Example 22

Preparation of carbonic acid, 1-[5,6-bis(4-methylphenyl)-1,2,4-triazin-3-yl]-4-piperidinyl phenyl ester

Following the procedure in Example 10, 15.0 g. (0.041 m.) of 1-[5,6-bis(4-methylphenyl)-1,2,4-triazin-3-yl]-4-piperidinol were reacted with 12.8 g. (0.082 m.) of phenyl chloroformate to give 11.3 g. (57.9% yield) of the desired product, m.p. 141.5—143°C.

Analysis: $C_{29}H_{28}N_4O_3$;
Calc: C, 72.48; H, 5.87; N, 11.66;
Found: C, 72.27; H, 6.07; N, 11.38.

Examples 23—25

Preparation of carbamate derivatives

Carbamate derivatives were prepared by direct aminolysis. The product from Example 22 (5.54 g., 0.011 m.) was stirred overnight with 100 ml. of anhydrous ammonia in 80 ml. of ethanol. The solution was then evaporated, and the residue dissolved in ether. The ether solution was washed with water, with 0.1N sodium hydroxide, with water, and then dried over anhydrous sodium sulfate and evaporated. Crystallization from ethyl acetate/Skelly B gave pure 1-[5,6-bis(4-methylphenyl)-1,2,4-triazin-3-yl]-4-piperidinol, carbamate (ester), m.p. 183—185°C.

Analysis: $C_{23}H_{25}N_5O_2$;
Calc: C, 68.47; H, 6.25; N, 17.36;
Found: C, 68.25; H, 6.45; N, 17.15.

Repeating Example 23, but using methylamine instead of ammonia, 1-[5,6-bis(4-methylphenyl)-1,2,4-triazin-3-yl]-4-piperidinol, N-methyl carbamate (ester) was obtained, m.p. 138—139.5°C.

Analysis: $C_{24}H_{27}N_5O_2$;
Calc: C, 69.04; H, 6.52; N, 16.77;
Found: C, 68.80; H, 6.51; N, 16.54.

Repeating Example 23, but using dimethylamine instead of ammonia, 1-[5,6-bis(4-methylphenyl-1,2,4-triazin-3-yl]-4-piperidinol, N,N-dimethylcarbamate (ester) was obtained, m.p. 162—163.5°C.

Analysis: $C_{25}N_{29}N_5O_2$;
Calc: C, 69.58; H, 6.77; N, 16.23;
Found: C, 69.50; H, 7.03; N, 15.93.

Example 26

Preparation of 1-[5,6-bis(4-methylphenyl)-1,2,4-triazin-3-yl]-4-piperidinol, methane sulfonate (ester)

Repeating Example 10, but using methanesulfonyl chloride in place of pivaloyl chloride, the title product was obtained, m.p. 174—177°C.

Analysis: $C_{23}H_{26}N_4O_3S$;
Calc: C, 62.99; H, 5.98; N, 12.78;
Found: C, 62.87; H, 5.73; N, 12.54.

Example 27

Preparation of 1-[5,6-bis(4-methylphenyl)-1,2,4-triazin-3-yl]-4-piperidinol, 4-methylbenzene sulfonate (ester)

Repeating Example 10, but using p-toluene-sulfonyl chloride in place of pivaloyl chloride, the title product was obtained, m.p. 132—134°C.

Analysis: $C_{29}H_{30}N_4O_3S$;
Calc: C, 67.68; H, 5.88; N, 10.89;
Found: C, 67.41; H, 5.62; N, 10.68.

Example 28

Preparation of 1-[5,6-bis(4-methylphenyl)-1,2,4-triazin-3-yl]-4-piperidinol, formate (ester)

Ten grams (0.02 m.) of 1-[5,6-bis(4-methylphenyl)-1,2,4-triazine-3-yl]-4-piperidinol were heated to 95°C. in 50 ml. of 98% formic acid for 50 minutes. Work up of the reaction mixture following the procedure in Example 1 and crystallization from Skelly B/ethyl acetate gave 6.8 g. (65.3% yield) of the desired product, m.p. 119—121°C.

Analysis: $C_{23}H_{24}N_4O_2$;
Calc: C, 71.11; H, 6.23; N, 14.42;
Found: C, 70.91; H, 6.40; N, 14.22.

Example 29

Preparation of 1-[5,6-bis(4-methylphenyl)-1,2,4-triazin-3-yl]-4-piperidinecarboxylic acid, ethyl ester

Following the procedure in Example 12, but using ethyl isonipecotate in place of 3-pyrrolidinol, the title product was obtained, m.p. 102—104°C.

Analysis: $C_{25}H_{28}N_4O_2$;
Calc: C, 72.09; H, 6.79; N, 13.45;
Found: C, 71.84; H, 6.61; N, 13.28.

Example 30

Preparation of 1-[5,6-bis(4-methylphenyl)-1,2,4-triazin-3-yl]-4-piperidinecarboxamide

Following the procedure in Example 12, but using isonipecotamide in place of 3-pyrrolidinol, the title product was obtained, m.p. 222—223.5°C.

Analysis: $C_{23}H_{35}N_5O$;
Calc: C, 71.29; H, 6.50; N, 18.07;
Found: C, 71.57; H, 6.69; N, 17.80.

Example 31

Preparation of 1-[5,6-bis(4-methylphenyl)-1,2,4-triazin-3-yl]-4-piperidinemethanol

Following the procedure in Example 1, but using 4-piperidine carbinol in place of 4-hydroxypiperidine, the desired product was prepared, m.p. 152—153.5°C.

Analysis: $C_{23}H_{26}N_4O$;
Calc: C, 73.77; H, 7.00; N, 14.96;
Found: C, 73.52; H, 7.22; N, 14.68.

Example 32

Preparation of 8-[5,6-bis(4-methylphenyl)-1,2,4-triazin-3-yl]-1,4-dioxa-8-azaspiro(4.5)decane

Following the procedure in Example 12, but using 1,4-dioxa-8-azaspiro[4.5]decane in place of 3-pyrrolidinol, gave the desired product, m.p. 169—170°C.

Analysis: $C_{24}H_{26}N_4O_2$;
Calc: C, 71.62; H, 6.51; N, 13.92;
Found: C, 71.34; H, 6.50; N, 13.79.

Example 33

Preparation of 1-[5,6-bis(4-methylphenyl)-1,2,4-triazin-3-yl]-4-piperidinone

Treatment of 2.0 g. (0.005 m.) of 8-[5,6-bis(4-methylphenyl)-1,2,4-triazin-3-yl)-1,4-dioxa-8-azaspiro[4.5]decane with 15 ml. of 90% formic acid for 15 minutes, evaporation of the solution and crystallization of the residue from isopropyl alcohol gave 1.5 g. (84.2% yield) of the desired product, m.p. 197—199°C.

Analysis: $C_{22}H_{22}N_4O$;
Calc: C, 73.72; H, 6.19; N, 15.63;
Found: C, 74.00; H, 6.22; N, 15.53.

Example 34

Preparation of 1-[5,6-bis(4-methylphenyl)-1,2,4-triazin-3-yl]-4-piperidinone, oxime

The piperidinone derivative prepared as in Example 33 (18 g., 0.05 m.) was slurried in 100 ml. of ethanol. The slurry was slowly added to a solution of 3.64 g. (0.052 m.) of hydroxylamine hydrochloride, 2.12 g. (0.052 m.) of sodium hydroxide, 10 ml. of water, and 100 ml. of ethanol. After heating at 50°C. for 3 hours, the reaction mixture was cooled. The resulting precipitate was filtered off and crystallized first from ethanol, then from ethyl acetate, giving the desired product, m.p. 179—181°C.

Analysis: $C_{22}H_{23}N_5O$;
Calc: C, 70.76; H, 6.21; N, 18.75;
Found: C, 70.55; H, 5.99; N, 18.93.

Example 35

Preparation of 1-[5,6-bis(4-methylphenyl)-pyrazin-2-yl]-4-piperidinol

13.1 g. (0.044 m.) of 2-chloro-5,6-bis(4-methylphenyl)-pyrazine were heated to reflux in 200 ml. of toluene and 8.9 g. (0.088 m.) of 4-hydroxypiperidine for three days. After high pressure liquid chromatography (Waters Prep 500, Silica gel eluting with ethyl acetate), 6.5 g. (41.4% yield) of the title compound were recovered, m.p. 176—178°C.

Analysis: $C_{23}H_{25}N_3O$;
Calc: C, 76.85; H, 7.01; N, 11.69;
Found: C, 76.66; H, 7.01; N, 11.42.

Example 36

Preparation 1-[5,6-bis(4-methylphenyl)-pyrazin-2-yl]-4-piperidinol, acetate (ester)

The product from Example 35 (2.6 g., 0.0072 m.) was acetylated following the procedure of Example 6. Crystallization from ethanol-water gave the desired product, m.p. 149—149.5°C.

Analysis: $C_{25}H_{27}N_3O_2$;
Calc: C, 74.79; H, 6.78; N, 10.42;
Found: C, 74.55; H, 6.97; N, 10.20.

The novel compounds of this invention were examined for their *in vitro* ability to activate GABA and benzodiazepine (BZ) binding. To measure the effect of a compound on GABA binding, Triton X-100

(octylphenoxy polyethoxyethanol, Rohm and Haas Co.) treated membrane protein was incubated in the presence of the compound and [$^3$H]GABA as detailed by Horng and Wong, *J. Neurochemistry, 32* (5), 1379 (1979). To examine BZ binding, $^3$H-flunirazepam and the compounds were incubated with native membrane protein as reported by Wong, et al, *Brain Res. Bull., 5* (Suppl. 2), 853 (1980). The results in Table 1 are the nanomolar concentrations of compounds (by Example No.) which produced a 50 percent increase ($SC_{50}$) in GABA or BZ binding. Each result is the average of one or more tests.

TABLE 1

*In vitro* activation of GABA and benzodiazepine (BZ) binding*

| Compound of Example No. | $SC_{50}$** GABA | BZ |
|---|---|---|
| 1 | 900 | 700 |
| 2 | >10,000 | 3500 |
| 3 | >10,000 | >10,000 |
| 4 | NT | >10,000 |
| 5 | >10,000 | 4000 |
| 6 | 3.3 | 5.5 |
| 7 | 15 | 12 |
| 8 | 60 | 1000 |
| 9 | 10 | 19 |
| 10 | 110 | 14 |
| 11 | 500 | 1000 |
| 12 | 5000 | >10,000 |
| 13 | 43 | 60 |
| 14 | 5200 | 515 |
| 15 | 2000 | 600 |
| 16 | 875 | 1138 |
| 17 | 400 | 700 |
| 18 | 160 | 180 |
| 19 | 70 | 18 |
| 20 | 80 | 90 |
| 21 | 10 | 21 |
| 22 | 160 | 650 |
| 23 | 56 | 15 |
| 24 | 100 | 24 |
| 25 | 1 | 3 |

TABLE 1 continued

| Compound of Example No. | $SC_{50}$** GABA | BZ |
|---|---|---|
| 26 | 68 | 6 |
| 27 | 8000 | 1200 |
| 28 | 13 | 10 |
| 29 | 12 | 18 |
| 30 | 800 | 8500 |
| 31 | 160 | 520 |
| 32 | >10,000 | 8000 |
| 33 | 200 | 430 |
| 34 | 1300 | 1600 |
| 35 | 1200 | 400 |
| 36 | 7 | 10 |

* For experimental detail, see text, Horng and Wong, *J. Neurochemistry, 32* (5), 1379 (1979), and Wong, et. al., *Brain Res. Bull., 5* (Suppl. 2) 853 (1980).
** Nanomolar concentration producing a 50% increase in binding
NT = Not tested.

Selected compounds were tested in the *in vivo* systems as described below and as summarized in Table 2.

Metrazole Induced Convulsion Inhibition Assay

In this assay, a compound to be tested was suspended in acacia (5%) and administered by gavage to each of three Cox standard strain albino male mice (18—24 grams) at the dose level being investigated. One hour after the oral administration, a water solution of metrazole (pentylenetetrazole) was administered by the intraperitoneal route to each mouse at a dose of 110 mg/kg. The mice were observed for one hour, during which time they were evaluated as to the degree of the metrazole induced convulsion. A score of 0 was assigned to a mouse not showing any signs of convulsant activity; a score of 1 was given to mice developing clonic convulsions; a score of 2 was given to mice showing flexor tonic convulsions; a score of 3 was given to mice showing extensor tonic convulsions; and a score of 4 was given to mice that died within the one hour. The scores of the three mice were totalled for each compound and dose level. The scores can range from 0 to 12; a score of 6 or less was interpreted as indicative of an active compound at that dose level. Values reported in Table 2 below are the minimum oral dose levels (mg./kg.) where activity as defined above was observed. For comparison purposes, it was found that 95% of saline or acacia-treated controls die under the above conditions. Diazepam shows activity in this test at about 1 mg/kg.

Electroshock Induced Convulsion
Inhibition Assay

The drug administration and test conditions used in this assay were similar to those used in the metrazole induced convulsion inhibition assay above except that a 0.1 second, 50 milliampere electroshock through corneal electrodes induced the convulsion instead of metrazole. The animals were examined and evaluated immediately after the electroshock and were scored as before. The results in Table 2 are expressed as the lowest dose of each compound tested which was active as previously defined. For comparison, 18 milliamperes was usually sufficient to produce extensor tonic convulsions in about half of the control animals; at 50 milliamperes, almost all control animals died. Diazepam was active at about 1 mg./kg.

Appetite Suppression Assay

In this test, groups of three Cox standard strain albino male mice were weighed (18—24 grams) and then fasted overnight (16—18 hours). The mice were reweighed and then given the test compound in a suspension of acacia (5%) by gavage. Thirty minutes after oral administration, the mice were allowed to eat freely for one hour. After the eating period, the mice were weighed once more. The percent of weight

gained (from the time the mice were given the test compound to after the feeding period) compared to weight lost during the fast period was calculated. The non-drug treated control animals gained back 35—55% of the weight lost on fasting. Any compound at the dose level tested which produced a weight gain of less than 10% was considered to be active. The results in Table 2 report the lowest dose level in which there was activity for each compound tested. Dextroamphetamine sulfate is active in this system at about 2.5 mg./kg.

Mouse Writhing Inhibition Assay

Writhing, which is characterized by contraction of the abdominal musculature, extension of the hindlegs, and rotation of the trunk, was induced in Cox standard strain albino male mice. The mice, weighing 18—24 grams, was fasted overnight and given the test compound by gavage in an acacia suspension (5%) 60 minutes before writhing was induced by the intraperitoneal administration of 55 mg./kg. of acetic acid (0.55 percent). Each treatment group consisted of 3 mice. The total number of writhes for the treatment group was determined during a 5-minute observation starting 5 minutes after acetic acid administration. Control groups had a total of 30—40 writhes per observation period. A compound which reduced the number of writhes to less than ten was considered active at that dose level. The results in Table 2 report the lowest dose level in which there was activity for each compound tested. Aspirin (acetyl salicylic acid) is active in this sytem at 200 mg./kg.

TABLE 2

*In vivo* testing of 5,6-bisaryl-triazine and pyrazine derivatives

| Compound of Example No. | mg./kg.* Metrazole | Electro-Shock | Appetite Suppression | Mouse Writhing |
|---|---|---|---|---|
| 1 | 3.1 | <25 | 3.1 | 3.1 |
| 5 | 12.5 | >25 | 25 | >25 |
| 6 | 6.25 | 25 | 12.5 | 12.5 |
| 7 | 50 | >50 | >50 | >50 |
| 8 | 200 | >200 | 200 | >200 |
| 9 | 12.5 | 12.5 | 12.5 | 12.5 |
| 11 | >200 | >200 | >200 | >200 |
| 12 | >200 | 200 | 12.5 | 200 |
| 14 | 12.5 | 50 | 12.5 | 12.5 |
| 15 | >200 | >200 | >200 | >200 |
| 17 | 50 | 50 | 12.5 | 12.5 |
| 18 | >200 | >200 | >200 | >200 |
| 19 | >200 | >200 | 50 | >200 |
| 20 | 6.2 | 6.2 | 6.2 | 6.2 |
| 22 | >200 | >200 | >200 | >200 |
| 23 | >6.25 | >6.25 | 0.4 | 1.56 |
| 26 | >6.25 | >6.25 | 0.4 | 0.4 |
| 27 | >200 | >200 | >200 | >200 |
| 29 | >200 | >200 | >200 | >200 |
| 32 | >200 | >200 | >200 | >200 |
| 33 | 50 | 50 | 12.5 | 50 |
| 35 | 12.5 | 50 | 12.5 | 12.5 |
| 36 | 25 | 100 | 25 | 100 |

* Minimum active oral dose. Refer to text for a description of the test systems.

**Claims for the Contracting States: BE CH DE FR GB IT LI LU NL SE**

1. A compound of the formula (I):

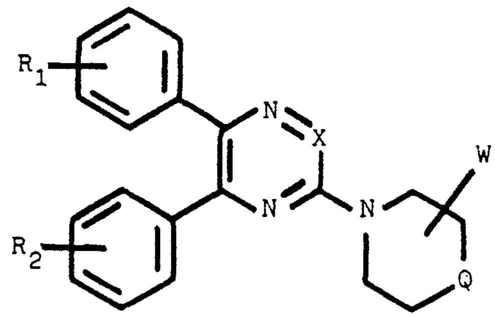

(I)

wherein

$R_1$ and $R_2$ are independently $C_1$—$C_3$ alkyl or chloro;

X is CH or N;

Q is oxygen or —$(CH_2)_n$—, wherein n is 0, 1, or 2;

W is a monovalent radical selected from hydrogen, $C_1$—$C_3$ alkyl, $C_1$—$C_3$ alkoxy, hydroxy, hydroxymethyl, —OCHO, —OCOA, —$OSO_2A$, or —COB, where A is $C_1$—$C_4$ alkyl, phenyl, phenoxy, amino, $C_1$—$C_3$ alkyl substituted phenyl, or mono- or di—$C_1$—$C_3$ alkylamino, and where B is $C_1$—$C_3$ alkoxy, amino, or mono- or di-$C_1$—$C_3$ alkylamino, or W is a divalent radical selected from =O, =NOH, or

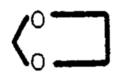

and pharmaceutically acceptable salt thereof.

2. A compound of claim 1 wherein $R_1$ and $R_2$ are both 4-chloro or are both 4-methyl.

3. A compound of claim 1 or claim 2 wherein Q is —$(CH_2)_n$—.

4. A compound of any one of claims 1—3 wherein X is N.

5. A compound of any one of claims 1—4 wherein W is hydroxy or —OCOA.

6. A compound of claim 1 which is 1-[5,6-bis(4-methylphenyl)-1,2,4-triazin-3-yl]-4-piperidinol,
1-[5,6-bis(4-chlorophenyl)-1,2,4-triazin-3-yl]-4-piperidinol,
1-[5,6-bis(4-methylphenyl)-1,2,4-triazin-3-yl]-4-piperidinol, acetate (ester),
1-[5,6-bis(4-methylphenyl)-1,2,4-triazin-3-yl]-4-piperidinol, propanoate (ester),
5,6-bis(4-methylphenyl)-3-(1-piperidinyl)-1,2,4-triazine,
1-[5,6-bis(4-methylphenyl)-1,2,4-triazin-3-yl]-3-piperidinol,
3-(4-methoxy-1-piperidinyl)-5,6-bis(4-methylphenyl)-1,2,4-triazine,
1-[5,6-bis(4-methylphenyl)-1,2,4-triazin-3-yl]-4-piperidinol, carbamate (ester),
1-[5,6-bis(4-methylphenyl)-1,2,4-triazin-3-yl]-4-piperidinol, methane sulfonate (ester),
1-[5,6-bis(4-methylphenyl)-1,2,4-triazin-3-yl]-4-piperidinone,
1-[5,6-bis(4-methylphenyl)-pyrazin-2-yl]-4-piperidinol or
1-[5,6-bis(4-methylphenyl)-pyrazin-2-yl)-4-piperidinol, acetate (ester)

7. A process for preparing a compound of the formula (I):

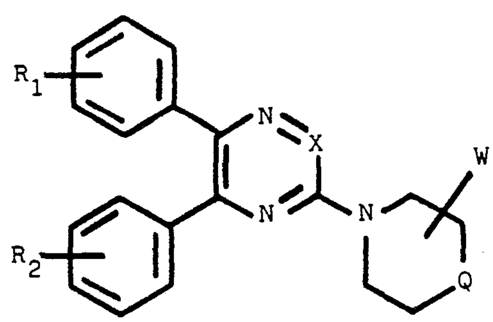

(I)

which comprises:

(a) reacting a precursor compound of formula (II):

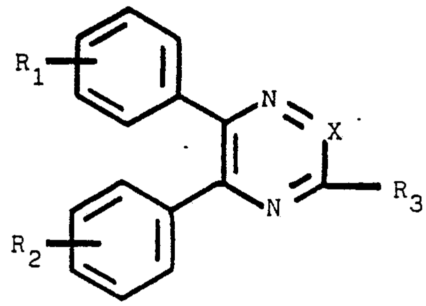

(II)

wherein $R_1$, $R_2$, and X are as defined above, and $R_3$ is a leaving group capable of undergoing nucleophilic displacement, with an amine of formula (III):

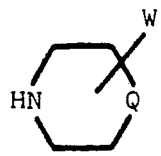

(III)

wherein W and Q are as defined above, and if appropriate optionally alkylating or esterifying a product of formula (I) in which W is hydroxyl; or

(b) reacting a compound of formula (I) in which W is a group of formula:

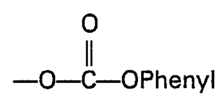

with ammonia or an amine of formula $HNR^{17}R^{18}$ where $R^{17}$ is $C_1$—$C_3$ alkyl and $R^{18}$ is hydrogen or $C_1$—$C_3$ alkyl so as to prepare a compound of formula (I) in which W is a group of formula —OCOA where A is amino or a mono- or di-$C_1$—$C_3$ alkylamino group.

8. A compound as claimed in any one of claims 1—6 for use as a pharmaceutical.

9. A compound as claimed in any one of claims 1—6 for use in treating mammals to modify their GABA neuronal function.

10. A pharmaceutical composition which comprises a compound of formula (I), stated in claim 1, or a pharmaceutically acceptable salt thereof associated with a pharmaceutically-acceptable carrier therefor.

**Claims for the Contracting State: AT**

1. A process for preparing a compound of the formula (I):

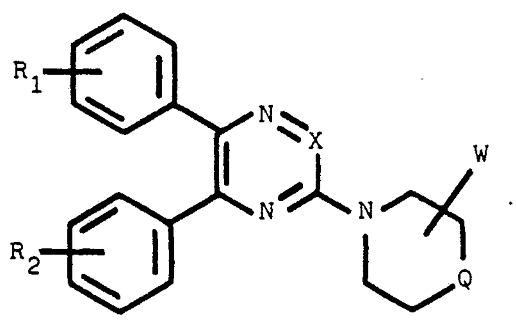

(I)

wherein

$R_1$ and $R_2$ are independently $C_1$—$C_3$ alkyl or chloro;

X is CH or N;

Q is oxygen or $-(CH_2)_n-$, wherein n is 0, 1, or 2;

W is a monovalent radical selected from hydrogen, $C_1$—$C_3$ alkyl, $C_1$—$C_3$ alkoxy, hydroxy, hydroxymethyl, —OCHO, —OCOA, —$OSO_2$A, or —COB, where A is $C_1$—$C_4$ alkyl, phenyl, phenoxy, amino, $C_1$—$C_3$ alkyl substituted phenyl, or mono- or di—$C_1$—$C_3$ alkylamino, and where B is $C_1$—$C_3$ alkoxy, amino, or moño- or di-$C_1$—$C_3$ alkylamino, or W is a divalent radical selected from =O, =NOH, or

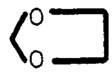

and pharmaceutically acceptable salt thereof, which comprises:

(a) reacting a precursor compound of formula (II):

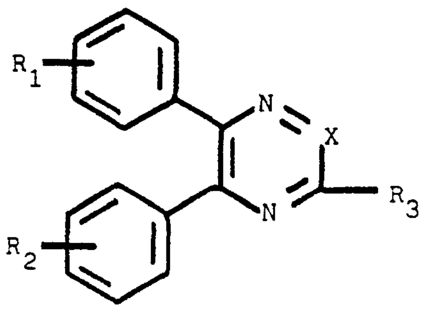

(II)

wherein $R_1$, $R_2$, and X are as defined above, and $R_3$ is a leaving group capable of undergoing nucleophilic displacement, with an amine of formula (III):

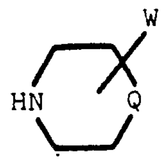

(III)

wherein W and Q are as defined above, and if appropriate optionally alkylating or esterifying a product of formula (I) in which W is hydroxyl; or

(b) reacting a compound of formula (I) in which W is a group of formula:

$$-O-\overset{\overset{O}{\|}}{C}-OPhenyl$$

with ammonia or an amine of formula $HNR^{17}R^{18}$ where $R^{17}$ is $C_1$—$C_3$ alkyl and $R^{18}$ is hydrogen or $C_1$—$C_3$ alkyl so as to prepare a compound of formula (I) in which W is a group of formula —OCOA where A is amino or a mono- or di-$C_1$—$C_3$ alkylamino group.

2. The process of claim 1 wherein $R_1$ and $R_2$ are 4-chloro.

3. The process of claim 1 wherein $R_1$ and $R_2$ are 4-methyl.

4. The process of any one of claims 1—3 wherein Q is $-(CH_2)_n-$.

5. The process of any one of claims 1—3 wherein X is N.

6. The process of any one of claims 1—5 wherein W is hydroxy.

7. The process of any one of claims 1—5 wherein W is —OCOA.

8. A compound of formula (I), or a pharmaceutically acceptable salt thereof whenever prepared by the process of any one of claim 1—7.

**Patentansprüche für die Vertragsstaaten: BE CH DE FR GB IT LI LU NL SE**

1. Verbindung der Formel (I)

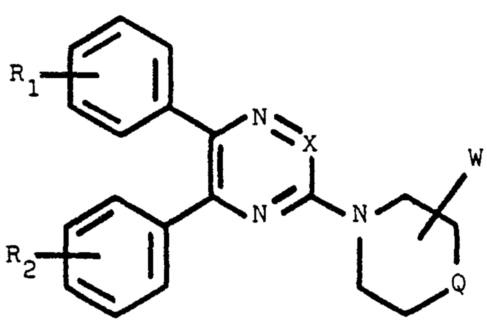

(I)

worin

$R_1$ und $R_2$ unabhängig $C_1$—$C_3$-Alkyl oder Chlor sind,

X für CH oder N steht,

Q Sauerstoff oder $-(CH_2)_n-$ ist, worin n für 0, 1 oder 2 steht,

W entweder ein einwertiger Rest ausgewählt aus Wasserstoff, $C_1-C_3$-Alkyl, $C_1-C_3$-Alkoxy, Hydroxy, Hydroxymethyl, —OCHO, —OCOA, $-OSO_2A$ oder —COB ist, worin A für $C_1-C_4$-Alkyl, Phenyl, Phenoxy, Amino, durch $C_1-C_3$-Alkyl substituiertes Phenyl oder Mono- oder Di-$C_1-C_3$-alkylamino steht und B für $C_1-C_3$-Alkoxy, Amino oder Mono- oder Di$C_1-C_3$-alkylamino steht, oder worin W ein zweiwertiger Rest ausgewählt aus =O, =NOH oder

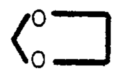

ist,

und die pharmazeutisch annehmbaren Salze hiervon.

2. Verbindung nach Anspruch 1, dadurch gekennzeichnet, daß $R_1$ und $R_2$ jeweils 4-Chlor oder 4-Methyl sind.

3. Verbindung nach Anpsruch 1 oder 2, dadurch gekennzeichnet, daß $Q-(CH_2)_n-$ ist.

4. Verbindung nach irgendeinem der Ansprüche 1 bis 3, dadurch gekennzeichnet, daß X für N steht.

5. Verbindung nach irgendeinem der Ansprüche 1 bis 4, dadurch gekennzeichnet, daß W Hydroxy oder —OCOA ist.

6. Verbindungen nach Anpsruch 1, nämlich

1-[5,6-Bis(4-methylphenyl)-1,2,4-triazin-3-yl]-4-piperidinol,
1-[5,6-Bis(4-chlorphenyl)-1,2,4-triazin-3-yl]-4-piperidinol,
1-[5,6-Bis(4-methylphenyl)-1,2,4-triazin-3-yl]-4-piperidinolacetat (Ester),
1-[5,6-Bis(4-methylphenyl)-1,2,4-triazin-3-yl]-4-piperidinolpropanoat (Ester),
5,6-Bis(4-methylphenyl)-3-(1-piperidinyl)-1,2,4-triazin,
1-[5,6-Bis(4-methylphenyl)-1,2,4-triazin-3-yl]-3-piperidinol,
3-[4-Methoxy-1-piperidinyl)-5,6-bis(4-methylphenyl)-1,2,4-triazin,
1-[5,6-Bis(4-methylphenyl)-1,2,4-triazin-3-yl]-4-piperidinolcarbamat (Ester),
1-[5,6-Bis(4-methylphenyl)-1,2,4-triazin-3-yl]-4-piperidinolmethansulfonat (Ester),
1-[5,6-Bis(4-methylphenyl)-1,2,4-triazin-3-yl]-4-piperidinon,
1-[5,6-Bis(4-methylphenyl)-pyrazin-2-yl]-4-piperidinol oder
1-[5,6-Bis(4-methylphenyl)-pyrazin-2-yl]-4-piperidinolacetat (Ester).

7. Verfahren zur Herstellung einer Verbindung der Formel (I)

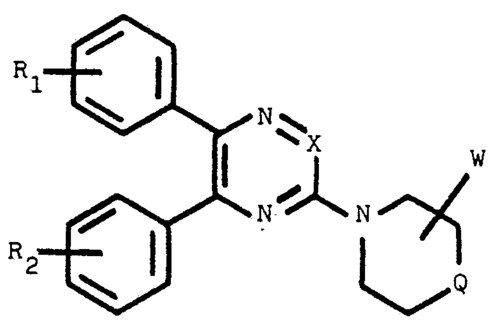

(I)

dadurch gekennzeichnet, daß man

(a) eine Vorläuferverbindung der Formel (II)

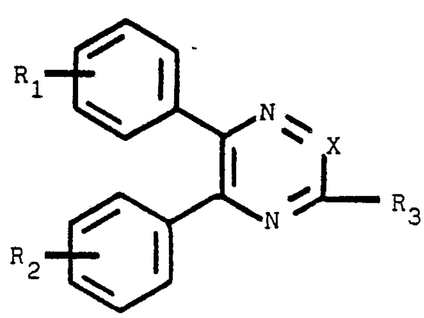

(II)

worin $R_1$, $R_2$ und X wie oben definiert sind und $R_3$ eine Angangsgruppe ist, die durch eine nukleophile Reaktion ausgetauscht werden kann, mit einem Amin der Formel (III)

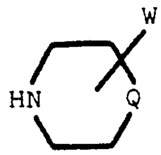

(III)

worin W und Q wie oben definiert sind, umsetzt und erforderlichenfalls wahlweise ein Produkt der Formel (I), worin W für Hydroxyl steht, alkyliert oder verestert oder

(b) eine Verbindung der Formel (I), worin W eine Gruppe der Formel

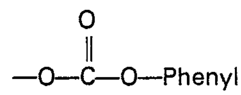

ist, mit Ammoniak oder einem Amin der Formel $HNR^{17}R^{18}$, worin $R^{17}$ für $C_1$—$C_3$-Alkyl steht und $R^{18}$ Wasserstoff oder $C_1$—$C_3$-Alkyl ist, zu einer Verbindung der Formel (I) umsetzt, worin W eine Gruppe der Formel —OCOA ist, in welcher A Amino oder eine Mono- oder Di-$C_1$—$C_3$-alkylaminogruppe bedeutet.

8. Verwendung einer Verbindung nach irgendeinem der Ansprüche 1 bis 6 als Pharmazeutikum.

9. Verwendung einer Verbindung nach irgendeinem der Ansprüche 1 bis 6 zur Behandlung von Säugetieren zur Abwandlung ihrer GABA-neuronalen Funktion.

10. Pharmazeutische Zusammensetzung , dadurch gekennzeichnet, daß sie eine Verbidnung der Formel (I) gamäß Anspruch 1 oder ein pharmazeutisch annehmbares Salz hiervon in Kombination mit einem pharmazeutisch annehmbaren Träger hierfür enthält.

**Patentansprüche für den Vertragsstaat: AT**

1. Verfahren zur Herstellung einer Verbindung der Formel (I)

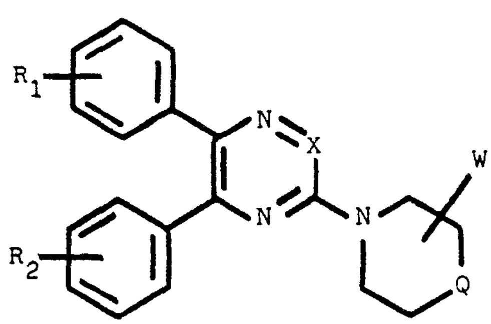

(I)

worin

$R_1$ und $R_2$ unabhängig $C_1$—$C_3$-Alkyl oder Chlor sind,

X für CH oder N steht,

Q Sauerstoff oder $—(CH_2)_n—$ ist, worin n für 0, 1 oder 2 steht,

W entweder ein einwertiger Rest ausgewählt aus Wasserstoff, $C_1$—$C_3$-Alkyl, $C_1$—$C_3$-Alkoxy, Hydroxy, Hydroxymethyl, —OCHO, —OCOA, $—OSO_2A$ oder —COB ist, worin A für $C_1$—$C_4$-Alkyl, Phenyl, Phenoxy, Amino, durch $C_1$—$C_3$-Alkyl substituiertes Phenyl oder Mono- oder Di-$C_1$—$C_3$-alkylamino steht und B für $C_1$—$C_3$-Alkoxy, Amino oder Mono- oder Di-$C_1$—$C_3$ alkylamino steht, oder worin W ein zweiwertiger Rest ausgewählt aus =O, =NOH oder

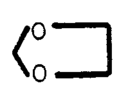

ist,

und der pharmazeutisch annehmbaren Salze hiervon, dadurch gekennzeichnet, daß man

(a) eine Vorläuferverbindung der Formel (II)

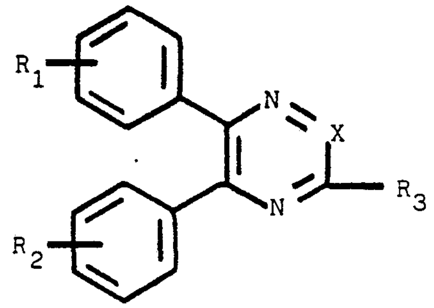

(II)

worin $R_1$, $R_2$ und X wie oben definiert sind und $R_3$ eine Abgangsgruppe ist, die durch eine nukleophile Reaktion ausgetauscht werden kann, mit einem Amin der Formel (III)

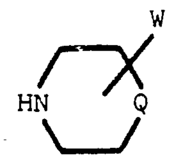

(III)

worin W und Q wie oben definiert sind, umsetzt und erforderlichenfalls wahlweise ein Produkt der Formel (I), worin W für Hydroxyl steht, alkyliert oder verestert oder

(b) eine Verbindung der Formel (I), worin W eine Gruppe der Formel

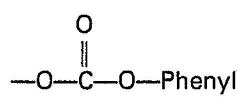

ist, mit Ammoniak oder einem Amin der Formel $HNR^{17}R^{18}$, worin $R^{17}$ für $C_1$—$C_3$-Alkyl steht und $R^{18}$ Wasserstoff oder $C_1$—$C_3$-Alkyl ist, zu einer Verbindung der Formel (I) umsetzt, worin W eine Gruppe der Formel —OCOA ist, in welcher A Amino oder eine Mono- oder Di-$C_1$—$C_3$-alkylaminogruppe bedeutet.

2. Verfahren nach Anspruch 1, dadurch gekennzeichnet, daß $R_1$ und $R_2$ für 4-Chlor stehen

3. Verfahren nach Anpsruch 1, dadurch gekennzeichnet, daß $R_1$ und $R_2$ für 4-Methyl stehen.

4. Verfahren nach irgendeinem der Ansprüche 1 bis 3, dadurch gekennzeichnet, daß Q für —$(CH_2)_n$— steht.

5. Verfahren nach irgenedeinem der Ansprüche 1 bis 3, dadurch gekennzeichnet, daß X für N steht.

6. Verfahren nach irgendeinem der Ansprüche 1 bis 5, dadurch gekennzeichnet, daß W Hydroxy ist.

7. Verfahren nach irgendeinem der Ansprüche 1 bis 5, dadurch gekennzeichnet, daß W für —OCOA steht.

8. Verbindung der Formel (I) oder ein pharmazeutisch annehmbares Salz hiervon, hergestellt nach dem in irgendeinem der Ansprüche 1 bis 7 beschriebenen Verfahren.

**Revendications pour les Etats contractants: BE CH LI FR GB IT LU NL SE DE**

1. Composé de formule (I):

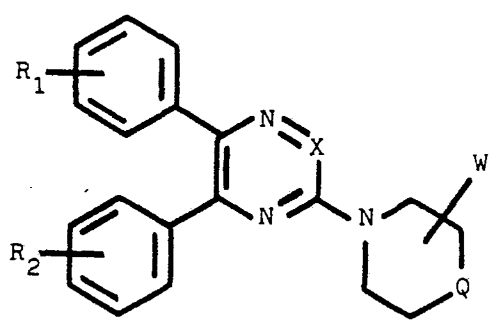

(I)

dans laquelle $R_1$ et $R_2$ représentent indépendamment l'un de l'auture un groupe alkyle en $C_1$—$C_3$ ou un atome de chlore;

X représente CH ou N;

Q représente un atome d'oxygène ou —$(CH_2)_n$— où n est égal à 0, 1 ou 2;

W représente un radical monovalent choisi parmi un atome d'hydrogène, un groupe alkyle en $C_1$—$C_3$,

un groupe alcoxy en $C_1$—$C_3$, un groupe hydroxy, un groupe hydroxy-méthyle, —OCHO, —OCOA, —$OSO_2A$ ou —COB où A représente un groupe alkyle en $C_1$—$C_4$, un groupe phényle, un groupe phénoxy, un groupe amino, un groupe phényle substitué par un groupe alkyle en $C_1$—$C_3$ ou un groupe mono- ou di-(alkyl en $C_1$—$C_3$)amino et B représente un groupe alcoxy en $C_1$—$C_3$, un groupe amino, un groupe mono- ou di-(alkyl en $C_1$—$C_3$)amino ou W représente un radical bivalent choisi parmi =O, =NOH ou

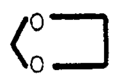

ainsi que ses sels pharmaceutiquement acceptables.

2. Composé selon la revendication 1, dans lequel $R_1$ et $R_2$ représentent chacun un groupe 4-chloro ou 4-méthyle.

3. Composé selon la revendication 1 ou 2, dans lequel Q représente —$(CH_2)_n$—.

4. Composé selon l'une quelconque des revendications 1 à 3, dans lequel X représente N.

5. Composé selon l'une quelconque des revendications 1 à 4, dans lequel W représente un groupe hydroxy ou —OCOA.

6. Composé selon la revendication 1, à savoir
le 1-[5,6-bis-(4-méthylphényl)-1,2,4-triazin-3-yl]-4-pipéridinol,
le 1-[5,6-bis-(4-chlorophényl)-1,2,4-triazin-3-yl]-4-pipéridinol,
le 1-[5,6-bis-(4-méthylphényl)-1,2,4-triazin-3-yl]-4-pipéridinol, acétate (ester),
le 1-[5,6-bis-(4-méthylphényl)-1,2,4-triazin-3-yl]-4-pipéridinol, propanoate (ester),
la 1-5,6-bis-(4-méthylphényl)-3-(1-pipéridinyl)-1,2,4-triazine,
le 1-5,6-bis-(4-méthylphényl)-1,2,4-triazin-3-yl]-3-pipéridinol,
la 3-(4-méthoxy-1-pipéridinyl)-5,6-bis-(4-méthylphényl)-1,2,4-triazine,
le 1-[5,6-bis-(4-méthylphényl)-1,2,4-triazin-3-yl]-4-pipéridinol, carbamate (ester),
le 1-[5,6-bis-(4-méthylphényl)-1,2,4-triazin-3-yl]-4-pipéridinol, méthane-sulfonate (ester),
la 1-[5,6-bis-(4-méthylphényl)-1,2,4-triazin-3-yl]-4-pipéridinone,
le 1-[5,6-bis-(4-méthylphényl)-pyrazin-2-yl]-4-pipéridinol, ou
le 1-[5,6-bis-(4-méthylphényl)-pyrazin-2-yl]-4-pipéridinol, acétate (ester).

7. Procédé de préparation d'un composé de formule (I):

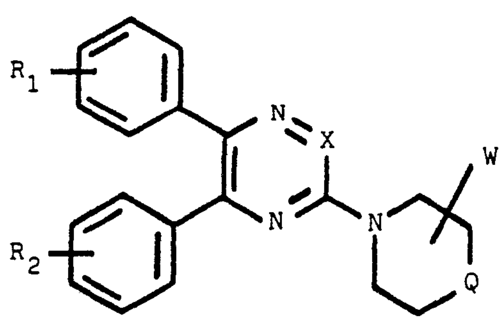

(I)

caractérisé en ce qu'il comprend les étapes qui consistent à:

(a) faire réagir un composé précurseur de formule (II):

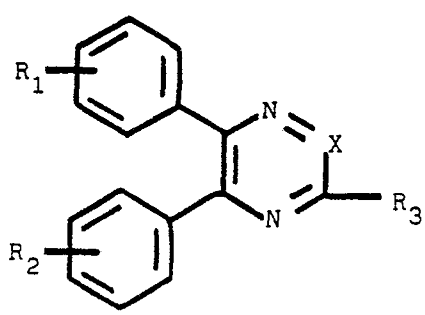

(II)

dans laquelle $R_1$, $R_2$ et X ont les significations définies ci-dessus et $R_3$ est un groupe qui s'éloigne, capable de subir un déplacement nucléophile, avec une amine de formule (III):

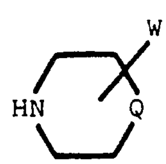

(III)

dans laquelle W et Q ont les significations définies ci-dessus et, si cela est approprié, éventuellement alkyler ou estérifier un produit de formule (I) dans laquelle W est un groupe hydroxy; ou

(b) faire réagir un composé de formule (I) dans laquelle W est un groupe de formule:

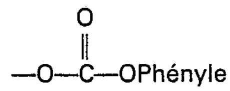

avec de l'ammoniac ou une amine de formule $HNR^{17}R^{18}$ où $R^{17}$ est un groupe alkyle en $C_1$—$C_3$ et $R^{18}$ est un atome d'hydrogène ou un groupe alkyle en $C_1$—$C_3$, de façon à préparer un composé de formule (I) dans laquelle W est un groupe de formule —OCOA où A est un groupe amino ou un groupe mono- ou di-(alkyl en $C_1$—$C_3$)amino.

8. Composé selon l'une quelconque des revendications 1 à 6, en vue de l'utiliser comme produit pharmaceutique.

9. Composé selon l'une quelconque des revendications 1 à 6, en vue de l'utiliser pour le traitement des mammifères afin de modifier leur fonction neuronale de GABA.

10. Composition pharamaceutique comprenant un composé de formule (I) selon la revendication 1 ou un de ses sels pharmaceutiquement acceptables en association avec un support pharmaceutiquement acceptable.

**Revendications pour l'Etat contractant: AT**

1. Procédé de préparation d'une composé de formule (I):

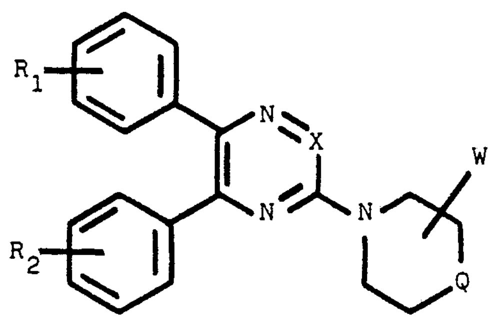

(I)

dans laquelle $R_1$ et $R_2$ représentent chacun indépendamment l'un de l'autre un groupe alkyle en $C_1$—$C_3$ ou un atome de chlore;

X représente CH ou N;

Q représente un atome d'oxygène ou $-(CH_2)_n-$ où n est égal à 0, 1 ou 2;

W représente un radical monovalent choisi parmi un atome d'hydrogène, un groupe alkyle en $C_1$—$C_3$, un groupe alcoxy en $C_1$—$C_3$, un groupe hydroxy, un groupe hydroxy-méthyle, —OCHO, —OCOA, $-OSO_2A$ ou —COB où A représente un groupe alkyle en $C_1$—$C_4$, un groupe phényle, un groupe phénoxy, un groupe amino, un groupe phényle substitué par un groupe alkyle en $C_1$—$C_3$ ou un groupe mono- ou di-(alkyl en $C_1$—$C_3$)amino et B représente un groupe alcoxy en $C_1$—$C_3$, un groupe amino ou un groupe mono- ou di-(alkyl en $C_1$—$C_3$)amino ou W représente un radical bivalent choisi parmi =O, =NOH ou

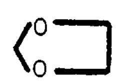

ainsi que de ses sels pharmaceutiquement acceptables, caractérisé en ce qu'il comprend les étapes qui consistent à:

(a) faire réagir un composé précurseur de formule (II):

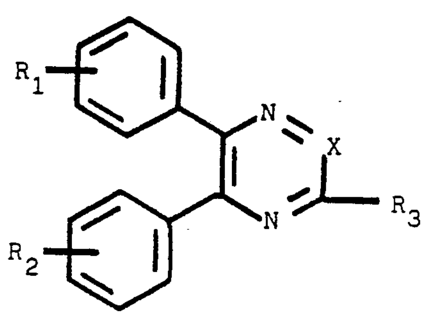

(II)

dans laquelle $R_1$, $R_2$ et X ont les significations définies ci-dessus et $R_3$ est un groupe qui s'éloigne, capable de subir un déplacement nucléophile, avec une amine de formule (III):

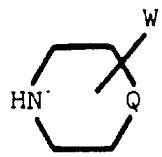

(III)

dans laquelle W et Q ont les significations définies ci-dessus et, si cela est approprié, éventuellement alkyler ou estérifier un produit de formule (I) dans laquelle W représente un groupe hydroxy; ou

(b) faire réagir un composé de formule (I) dans laquelle W est un groupe de formule:

$$-O-\overset{\overset{\displaystyle O}{\|}}{C}-O\text{Phényle}$$

avec de l'ammoniac ou une amine de formule $HNR^{17}R^{17}$ où $R^{17}$ est un groupe alkyle en $C_1$—$C_3$ et $R^{18}$ est un atome d'hydrogène ou un groupe alkyle en $C_1$—$C_3$, afin de préparer un composé de formule (I) dans laquelle W est un groupe de formule —OCOA où A est un groupe amino ou un groupe mono- ou di-(alkyl en $C_1$—$C_3$)amino.

2. Procédé selon la revendication 1, caractérisé en ce que $R_1$ et $R_2$ représentent chacun un groupe 4-chloro.

3. Procédé selon la revendication 1, caractérisé en ce que $R_1$ et $R_2$ représentent chacun un groupe 4-méthyle.

4. Procédé selon l'une quelconque des revendications 1 à 3 caractérisé en ce que Q représente $-(CH_2)_n-$.

5. Procédé selon l'une quelconque des revendications 1 à 3, caractérisé en ce que X représente N.

6. Procédé selon l'une quelconque des revendications 1 à 5, caractérisé en ce que W représente un groupe hydroxy.

7. Procédé selon l'une quelconque des revendications 1 à 5, caractérisé en ce que W représente —OCOA.

8. Composé de formule (I) ou un de ses sels pharamceutiquement acceptables, que l'on prépare par le procédé selon l'une quelconque des revendications 1 à 7.